# EUROPEAN PATENT APPLICATION

(11) **EP 4 635 506 A1**
(43) Date of publication of application: **22.10.2025**
(21) Application number: 23903703.9
(22) Date of filing: 13.10.2023
(51) Int. Cl.: A61K 35/51, A61P 15/08

(54) **PHARMACEUTICAL COMPOSITION FOR PREVENTING OR TREATING OVARIAN FAILURE COMPRISING UMBILICAL CORD-DERIVED ADHERENT STEM CELLS**

(30) Priority: 13.12.2022 KR 20220173666; 07.09.2023 KR 20230119165
(71) Applicant: Cha Biotech Co., Ltd., Gyeonggi-do 13488 (KR)
(72) Inventor: YU, Ji Min, Seongnam-si, Gyeonggi-do 13602 (KR); KIM, Hyun Ji, Seoul 08608 (KR); SONG, Chaeyoung, Yongin-si, Gyeonggi-do 16827 (KR); KIM, Kyung Min, Suwon-si Gyeonggi-do 16697 (KR); KIM, Ji Hyang, Seoul 05555 (KR)
(74) Representative: Weickmann & Weickmann PartmbB
(86) International application number: PCT/KR2023/015837
(87) International publication number: WO 2024/128499

(57) **Abstract**

The present disclosure relates to a pharmaceutical composition for preventing or treating ovarian insufficiency, including umbilical cord-derived adherent stem cells. In an embodiment, the umbilical cord-derived adherent stem cells may increase the number and proportion of follicles or primordial follicles, induce superovulation of oocytes, or promote the development of fertilized embryos. Accordingly, the composition may be usefully employed in the prevention or treatment of ovarian insufficiency, including premature menopause or infertility.

## Description

### Technical Field

The present disclosure relates to a pharmaceutical composition for preventing or treating ovarian insufficiency.

### Background

For women, age and pregnancy are closely related, and pregnancy rates tend to decline with increasing age. The primary causes of this phenomenon include a decrease in the number and quality of oocytes in the ovaries. In recent years, the average age at marriage has risen annually, and even in the absence of identifiable causes of infertility, age-related decline in ovarian function, specifically reduced oocyte count and quality, has become increasingly prevalent.

The ovary is a reproductive organ in women that produces oocytes and secretes sex hormones such as estrogen, progesterone, and testosterone. The ovary plays a critical role not only in maintaining the health of the female reproductive system but also in regulating hormonal balance, thereby contributing significantly to a woman's quality of life. Ovarian function can be assessed by measuring changes in levels of follicle-stimulating hormone (FSH), estradiol, or anti-Mullerian hormone (AMH).

Although assisted reproductive technologies such as in vitro fertilization and intracytoplasmic sperm injection have advanced in recent years, ovarian insufficiency remains essentially untreatable even with state-of-the-art reproductive medicine. Therefore, there is a need to develop means to suppress the decline in ovarian function.

### Disclosure of Invention

### Technical Problem

One aspect of the present disclosure provides a pharmaceutical composition for preventing or treating ovarian insufficiency, including umbilical cord-derived adherent stem cells, a cell population thereof, or a culture thereof as an active ingredient.

Another aspect provides a method of preventing or treating ovarian insufficiency, including administering an effective amount of umbilical cord-derived adherent stem cells, a cell population thereof, or a culture thereof to a subject in need thereof.

Another aspect provides a method of increasing the number of follicles or primordial follicles in a subject, including administering an effective amount of umbilical cord-derived adherent stem cells, a cell population thereof, or a culture thereof to the subject in need thereof.

Another aspect provides a method of promoting ovulation of oocytes in a subject, including administering an effective amount of umbilical cord-derived adherent stem cells, a cell population thereof, or a culture thereof to the subject in need thereof.

Another aspect provides a method of promoting development of fertilized embryos in a subject, including administering an effective amount of umbilical cord-derived adherent stem cells, a cell population thereof, or a culture thereof to the subject in need thereof.

Another aspect provides a method of increasing or decreasing the function or activity of cells that are decreased or increased, respectively, in ovarian insufficiency, including administering an effective amount of umbilical cord-derived adherent stem cells, a cell population thereof, or a culture thereof to a subject in need thereof.

Another aspect provides the use of umbilical cord-derived adherent stem cells, a cell population thereof, or a culture thereof for the prevention or treatment of ovarian insufficiency.

### Solution to Problem

One aspect of the present disclosure provides a pharmaceutical composition for preventing or treating ovarian insufficiency, including umbilical cord-derived adherent stem cells, a cell population thereof, or a culture thereof as an active ingredient.

Another aspect provides a method of preventing or treating ovarian insufficiency, including administering an effective amount of umbilical cord-derived adherent stem cells, a cell population thereof, or a culture thereof to a subject in need thereof.

Another aspect provides a method of increasing the number of follicles or primordial follicles in a subject, including administering an effective amount of umbilical cord-derived adherent stem cells, a cell population thereof, or a culture thereof to the subject in need thereof.

Another aspect provides a method of promoting ovulation of oocytes in a subject, including administering an effective amount of umbilical cord-derived adherent stem cells, a cell population thereof, or a culture thereof to the subject in need thereof.

Another aspect provides a method of promoting development of fertilized embryos in a subject, including administering an effective amount of umbilical cord-derived adherent stem cells, a cell population thereof, or a culture thereof to the subject in need thereof.

Another aspect provides a method of increasing or decreasing the function or activity of cells that are decreased or increased, respectively, in ovarian insufficiency, including administering an effective amount of umbilical cord-derived adherent stem cells, a cell population thereof, or a culture thereof to a subject in need thereof.

In the present specification, the term "umbilical cord" may refer to the cord that connects a mammalian fetus to the mother, allowing the fetus to grow via the placenta. It may specifically refer to tissue composed of three blood vessels-two umbilical arteries and one umbilical vein-typically surrounded by Wharton's jelly. Accordingly, in the present specification, the term "umbilical cord-derived adherent stem cells" refers to cells derived from the umbilical cord or Wharton's jelly tissue thereof, which have the ability to differentiate into various tissue types and exhibit the characteristic of growing while adherent to the surface of a culture vessel.

The umbilical cord-derived adherent stem cells of the present specification are cells having the same cellular characteristics as those described in the examples of Korean Patent Application No. 10-2016-0102721, the entire disclosure of which is incorporated herein by reference.

The umbilical cord-derived adherent stem cells provided in the present specification may express CD200, CD141, CD61, CD87, or SSEA4 as positive surface markers on at least about 20 %, 25 %, 30 %, 35 %, 40 %, 45 %, 50 %, 55 %, 60 %, 65 %, 70 %, 75 %, 80 %, 85 %, 90 %, 95 %, 98 %, or 99 % of the cells. The umbilical cord-derived adherent stem cells provided in the present specification may express stem cell markers such as Oct4, Nanog, Tra-1-60, CD3, CD1a, CD11c, CD16, CD86, CD8a, MIC A/B, or CD40 as negative markers at a level of no more than about 70 %, no more than 60 %, no more than 50 %, no more than 40 %, no more than 30 %, no more than 20 %, no more than 10 %, no more than 5 %, or no more than 1 % of the cells. In particular, the cells of the present specification may express MIC A/B in no more than about 10 %, 5 %, or 1 % of the total cell population. In particular, the cells of the present specification may express SSEA4 in at least 80 %, 85 %, or 90 % of the total cell population. In particular, the cells of the present specification may express Oct4 and Nanog in no more than 10 %, 5 %, or 1 % of the total cell population. In particular, the cells of the present specification may express CD200 in at least 85 %, 90 %, or 95 % of the total cell population. Among the surface antigen characteristics, CD61+ may be a surface marker that is overexpressed under hypoxic conditions.

In the present disclosure, the term "positive" may refer to the presence of a marker, in the context of stem cell markers, in a greater amount or at a higher concentration relative to other non-stem cells used as a reference. That is, if a marker is present inside the cell or on the cell surface in such a way that it allows the cell to be distinguished from one or more other cell types, the cell is considered positive for that marker. The term may also mean that the cell expresses the marker in an amount sufficient to generate a detectable signal above background, such as a signal measurable by a cell analysis device. For example, if a cell can be labeled with an antibody specific for CD200 and the signal from that antibody is detectably greater than the control (e.g., background level), then the cell is "CD200+". In the present disclosure, the term "negative" means that the marker cannot be detected in comparison to the background level even when an antibody specific to a particular cell surface marker is used. For example, if a cell cannot be labeled with an antibody specific to CD3 in a detectable manner, then the cell is "CD3-".

The above-described immunological characteristics may be determined by conventional methods known in the technical field to which the present disclosure pertains. For example, various methods such as flow cytometry, immunohistochemical staining, or RT-PCR may be used.

In an embodiment, the umbilical cord-derived adherent stem cells may have one or more characteristics selected from (a) to (e) below:
a) increased expression, compared to bone marrow-derived stem cells, of one or more selected from the group consisting of COL1A1, IGFBP4, TAGLN, STC1, LRRC17, and IL33;
b) decreased expression, compared to bone marrow-derived stem cells, of one or more selected from the group consisting of CCND1, SERPINE1, PRNP, and CYP1B1;
c) maintenance of the morphology of adherent fibroblast-like cells during serial passaging;
d) the ability to differentiate into adipocytes, osteocytes, or chondrocytes; and
e) one or more surface antigen characteristics selected from the group consisting of CD200+, Tra-1-60-, CD3-, CD1a-, CD11c-, CD16-, CD86-, CD8a-, CD40-, CD141+, CD61+, CD87+, MIC A/B- and SSEA4+.

In an embodiment, the umbilical cord-derived adherent stem cells may further have one or more characteristics selected from (f) to (i) below:
f) increased expression, compared to culture under normoxic conditions, of one or more selected from the group consisting of S100A10, BNIP3, IGFBP5, NDUFA4L2, DPYD, and SCARA3;
g) decreased expression, compared to culture under normoxic conditions, of one or more selected from the group consisting of IL8, ALDH1A1, DLC1, CTHRC1, and CPA4;
h) increased expression, compared to bone marrow-derived stem cells, of one or more selected from the group consisting of SNCA, DSG2, NRP2, and PLAT; and
i) decreased expression, compared to bone marrow-derived stem cells, of one or more selected from the group consisting of TPMT, NAGK, and ANXA4.

The umbilical cord-derived adherent stem cells according to an embodiment may express, at higher levels than bone marrow-derived stem cells, one or more genes or proteins selected from the group consisting of COL1A1, IGFBP4, TAGLN, STC1, LRRC17, and IL33. Specifically, the cells may express two or more, or three or more, and more specifically, all of the genes or proteins selected from the group consisting of COL1A1, IGFBP4, TAGLN, STC1, LRRC17, and IL33 at higher levels than bone marrow-derived stem cells. In addition, the umbilical cord-derived adherent stem cells according to an embodiment may further include genes such as S100A10, SQSTM1, DSTN, DCN, PHGDH, FBLN1, MFGE8, HLA-A, VASN, or KIAA1199, which are expressed at higher levels compared to bone marrow-derived stem cells. These genes have not been previously reported in relation to umbilical cord-derived adherent stem cells. The umbilical cord-derived adherent stem cells according to an embodiment may exhibit at least a two-fold increase in expression levels of the above genes compared to bone marrow-derived stem cells. The difference in expression levels may be based on, for example, comparison of gene expression at the mRNA level. The difference in expression may also be determined, for example, through microarray analysis.

In addition, the umbilical cord-derived adherent stem cells according to an embodiment may express, at lower levels than bone marrow-derived stem cells, one or more genes or proteins selected from the group consisting of CCND1, SERPINE1, PRNP, and CYP1B1. Specifically, the cells may express two or more, or three or more, and more specifically, all of the genes or proteins selected from the group consisting of CCND1, SERPINE1, PRNP, and CYP1B1 at lower levels than bone marrow-derived stem cells. The genes or proteins that may be expressed at lower levels in the umbilical cord-derived adherent stem cells according to an embodiment, compared to bone marrow-derived stem cells, may include MTA2A, TM4SF1, HIST1H4C, and NME1. These genes or proteins have not previously been reported in association with umbilical cord-derived adherent stem cells. The umbilical cord-derived adherent stem cells according to an embodiment may exhibit at least a two-fold difference in the expression levels of the above genes or proteins compared to bone marrow-derived stem cells. The difference in expression levels may be based on, for example, comparison of gene expression at the mRNA level. In addition, the difference in expression may also be determined, for example, through microarray analysis.

In addition, the umbilical cord-derived adherent stem cells may exhibit a fibroblast-like morphology when serially passaged. In an embodiment, the cells may exhibit properties of adherent cells that require attachment to a surface for in vitro growth and may display a spindle-shaped fibroblastic morphology.

In another embodiment, the umbilical cord-derived adherent stem cells may possess colony-forming ability. The cells may exhibit a higher colony-forming efficiency compared to cells cultured under normoxic conditions.

Additionally, the umbilical cord-derived adherent stem cells may differentiate into adipocytes, osteocytes, or chondrocytes. For example, the cells may be induced to differentiate along specific cell lineages such as adipogenic, chondrogenic, osteoblastic, hematopoietic, myogenic, vascular, neurogenic, or hepatogenic lineages.

The umbilical cord-derived adherent stem cells may also differentiate into adipocytes, osteocytes, or chondrocytes. For example, the cells may be induced to differentiate along specific cell lineages, including but not limited to adipogenic, chondrogenic, osteoblastic, hematopoietic, myogenic, vascular, neurogenic, and hepatogenic differentiation.

The term "differentiation" refers to the process by which cells become specialized in structure or function as they grow and proliferate, that is, the phenomenon in which biological cells or tissues undergo changes in form or function in order to perform their designated roles. Differentiation into a specific cell type may be assessed using methods well known in the relevant field, and induction of differentiation into a particular cell type may be carried out using established techniques. The differentiation may be confirmed, for example, by analyzing changes in cell surface markers using techniques such as flow cytometry or immunocytochemistry (e.g., staining cells with tissue-specific or cell marker-specific antibodies), and by examining cellular morphology using optical or confocal microscopy. Additionally, changes in gene expression may be confirmed using techniques well known in the field, such as PCR or gene expression profiling.

In the present specification, the term "isolation of umbilical cord-derived adherent stem cells" may refer to the removal of at least 20 %, 30 %, 40 %, 50 %, 60 %, 70 %, 80 %, 90 %, 95 %, or 99 % of the cells that are normally associated with stem cells in untreated mammalian umbilical cords. A population of cells obtained from an organ may be considered "isolated" if, in the untreated state of that organ, other cells normally associated with the stem cells constitute less than 50 % of the total cell population.

The ovarian insufficiency may be any one selected from primary ovarian insufficiency, secondary ovarian insufficiency, hyperandrogenemia, polycystic ovary syndrome, amenorrhea, oligomenorrhea, premature menopause, and infertility.

In an embodiment, the stem cells may increase the number of follicles or primordial follicles.

In an embodiment, the stem cells may promote ovulation of oocytes.

In an embodiment, the stem cells may promote development of fertilized embryos.

In an embodiment, the stem cells may restore the function or activity of any one cell selected from the group consisting of B cells, dendritic cells, and fibroblasts, which are decreased in ovarian insufficiency.

In an embodiment, the stem cells may reduce the number or activity of luteinizing mural cells or atretic cells, which are increased in ovarian insufficiency.

In an embodiment, the stem cells may increase the expression or activity of Cd 19 or Cd 209a.

In an embodiment, the stem cells may increase the expression or activity of Itm2a, or decrease the expression or activity of Parm1 or Chf.

In an embodiment, the decreased cells in ovarian insufficiency may be any one cell selected from the group consisting of B cells, dendritic cells, and fibroblasts.

In an embodiment, the increased cells in ovarian insufficiency may be luteinizing mural cells or atretic cells.

In addition, the aspect may include a pharmaceutical composition including a culture medium of the umbilical cord-derived adherent stem cells. For example, the present specification provides a pharmaceutical composition including the umbilical cord-derived adherent stem cells, a cell population thereof, or a culture medium thereof as an active ingredient.

The dosage of the cell therapy or pharmaceutical composition according to an embodiment, based on umbilical cord-derived adherent stem cells, may be from 1.0 × 10³ to 1.0 × 10¹⁰ cells per kg (body weight) or per subject, or from 1.0 × 10⁷ to 1.0 × 10⁸ cells per kg (body weight) or per subject. However, the dosage may vary depending on factors such as the formulation method, route of administration, age, body weight, sex, pathological condition, diet, time of administration, route of administration, excretion rate, and responsiveness. One skilled in the art may appropriately adjust the dosage in consideration of such factors. The frequency of administration may be a single dose or two or more doses within the range of clinically acceptable adverse effects, and administration may be performed at one or more sites. For animals other than humans, the dosage may be the same as that for humans on a per kg or per subject basis, or may be adjusted based on, for example, organ volume ratios (e.g., average values) between the target animal and humans (such as heart volume). The subject animal to be treated according to an embodiment may include humans and other mammals intended for therapeutic purposes, specifically including humans, monkeys, mice, rats, rabbits, sheep, cattle, dogs, horses, and pigs.

The cell therapy or pharmaceutical composition according to an embodiment may include the umbilical cord-derived adherent stem cells as an active ingredient, together with a pharmaceutically acceptable carrier and/or additive. For example, the composition may include sterile water, physiological saline, conventional buffers (such as phosphate, citric acid, or other organic acids), stabilizers, salts, antioxidants (such as ascorbic acid), surfactants, suspending agents, tonicity agents, or preservatives. For topical administration, it is preferable to formulate the composition in combination with organic materials such as biopolymers, or inorganic materials such as hydroxyapatite. Specific examples include collagen matrices, polylactic acid polymers or copolymers, polyethylene glycol polymers or copolymers, and chemical derivatives thereof. When the cell therapy or pharmaceutical composition according to an embodiment is formulated for injection, the cell aggregate may be dissolved in a pharmaceutically acceptable carrier or may be in a frozen solution state containing the carrier.

The umbilical cord-derived adherent stem cells according to an embodiment may be used in various types of therapeutic protocols in which a target tissue or organ in the body is reinforced, treated, or replaced by the engraftment, transplantation, or injection of a desired cell population, such as stem cells or stem cell-derived cell populations. The umbilical cord-derived adherent stem cells may replace or enhance existing tissue, leading to new or modified tissue, or may be combined with biological tissue or structures. In therapeutic protocols where stem cells derived from tissues other than umbilical cord are typically used, the stem cells may be replaced with the umbilical cord-derived adherent stem cells described in the present specification.

The cell therapy or pharmaceutical composition according to an embodiment may, depending on the route of administration or dosage form, further include appropriate components such as suspending agents, solubilizing agents, stabilizers, tonicity agents, preservatives, anti-adsorption agents, surfactants, diluents, excipients, pH-adjusting agents, analgesics, buffers, reducing agents, or antioxidants, as necessary. Pharmaceutically acceptable carriers and formulations suitable for the present disclosure, including those listed above, are described in detail in [Remington's Pharmaceutical Sciences, 19th ed., 1995].

The cell therapy or pharmaceutical composition according to an embodiment may be formulated into a unit dosage form or placed in a multi-dose container using pharmaceutically acceptable carriers and/or excipients, in accordance with methods readily implementable by those skilled in the art to which the present disclosure pertains. The formulation may be in the form of a solution, suspension, or emulsion in an oil-based or aqueous medium, or in the form of a powder, granule, tablet, or capsule. The cell therapy may also be formulated as an injectable preparation. In such cases, conventionally known formulation components may be used, and the formulation may be prepared by standard methods.

### Advantageous Effects of Invention

According to one aspect, the umbilical cord-derived adherent stem cells may increase the number and proportion of follicles or primordial follicles in a subject, induce superovulation of oocytes, or promote the development of fertilized embryos. Accordingly, they may be usefully employed for the prevention or treatment of ovarian insufficiency, including premature menopause or infertility.

### Brief Description of Drawings

FIGS. 1 and 2 are graphs showing the estrous cycle in mice with POI induced by CP treatment.
FIG. 3 is a graph showing ovarian weight in mice with POI induced by CP treatment.
FIG. 4 is a histological image and graph showing the number and proportion of follicles in mice with POI induced by CP treatment.
FIG. 5 is a histological image showing the number and proportion of follicles and primordial follicles following administration of the stem cells according to an embodiment.
FIG. 6 is a graph measuring the number and proportion of follicles and primordial follicles following administration of the stem cells according to an embodiment.
FIG. 7 is a histological image showing ovarian apoptosis following administration of the stem cells according to an embodiment.
FIG. 8 is a graph measuring ovarian apoptosis following administration of the stem cells according to an embodiment.
FIG. 9 is a histological image showing Anti-Müllerian Hormone (AMH) staining area following administration of the stem cells according to an embodiment.
FIG. 10 is a graph measuring Anti-Müllerian Hormone (AMH) staining area following administration of the stem cells according to an embodiment.
FIG. 11 is a microscopic image showing the number of superovulated oocytes following administration of the stem cells according to an embodiment.
FIG. 12 is a graph measuring the number of superovulated oocytes following administration of the stem cells according to an embodiment.
FIG. 13 is a microscopic image showing the proportion of 2-cell and blastocyst-stage embryos during in vitro fertilization following administration of the stem cells according to an embodiment.
FIG. 14 shows the results of measuring the proportion of 2-cell and blastocyst-stage embryos during in vitro fertilization following administration of the stem cells according to an embodiment.
FIG. 15 is a graph showing the analysis results of immune cell populations present in the ovary.
FIG. 16 is a graph showing changes in immune cell populations following administration of the stem cells according to an embodiment.
FIG. 17 is a graph showing the analysis results of stromal cell populations present in the ovary.
FIG. 18 is a graph showing changes in stromal cell populations following administration of the stem cells according to an embodiment.
FIG. 19 is a graph showing the analysis results of granulosa cell populations present in the ovary.
FIG. 20 is a graph showing changes in granulosa cell populations following administration of the stem cells according to an embodiment.

### Best Mode for Carrying out the Invention

The following preferred examples are provided to aid in the understanding of the present disclosure. However, these examples are provided merely for illustrative purposes and are not intended to limit the scope of the present disclosure. Various modifications may be made to the examples, and the disclosure is not limited to the embodiments disclosed below but may be implemented in various other forms.

Example: Preparation and Characterization of Umbilical Cord-Derived Adherent Stem Cells

### 1.Preparation of Umbilical Cord-Derived Adherent Stem Cells

Umbilical cord-derived adherent stem cells were prepared in accordance with the same method as described in Korean Patent Application No. 10-2016-0102721, the entire disclosure of which is incorporated herein by reference in its entirety.

With informed consent obtained in advance based on sufficient explanation, umbilical cords were isolated from placental tissues collected at the time of normal delivery from healthy mothers. The isolated umbilical cords were washed 2 to 5 times with Ca/Mg-free DPBS to remove residual blood. Without removing the outer amniotic membrane, two arteries and one vein were excised, and the umbilical cord was cut into segments approximately 1 to 5 mm in length. These segments were then attached to culture vessels and incubated for 10 to 15 days. Upon confirmation of cell outgrowth from the cultured tissue, the tissue was treated with 200 U/mL of collagenase I for 5 to 6 hours to isolate the umbilical cord-derived adherent stem cells. Before and after the collagenase I treatment, cell morphology and outgrowth from the umbilical cord-attached tissue were confirmed using an optical microscope at 40× and 100× magnification. The isolated cells (designated as passage 0; P0) were then cultured in MEM alpha GlutaMAX medium (CS-CM medium) supplemented with 25 ng/mL FGF4, 1 µg/mL heparin, and 10 % FBS, under hypoxic conditions (3 % O₂) at 37 °C. The CS-CM medium was replaced every 3 to 4 days to remove non-adherent cells from the flask surface. For serial passaging at the first passage, the adherent cells were treated for a short period (3 minutes) at 37 °C with TrypLE, a recombinant, animal component-free (ACF) enzyme produced by Invitrogen. Hereinafter, the isolated stem cells are referred to as "CordSTEM".

### 2. Characterization of Umbilical Cord-Derived Adherent Stem Cells

The surface protein profile of the umbilical cord-derived adherent stem cells prepared in Example 1 was analyzed by flow cytometry and immunofluorescence staining. These analyses were conducted in the same manner as described in Korean Patent Application No. 10-2016-0102721, the entire disclosure of which is incorporated herein by reference.

Specifically, for flow cytometry, the cells were first washed with DPBS and then suspended in DPBS containing 2 % FBS. The cells were incubated on ice for 20 minutes with antibodies against the following markers: Tra-1-60, CD3, CD1a, CD11c, CD16, CD14, CD86, CD8a, CD19, CD40, CD80, CD200, CD141, CD61, CD87, MIC A/B, and SSEA4. Surface antigen expression was then analyzed using a FACS Calibur flow cytometer (Becton Dickinson). In addition, to confirm the expression of the embryonic stem cell markers Oct4 and Nanog, immunofluorescence staining was performed. First, the cells were washed three times with DPBS and fixed in 4 % paraformaldehyde in the culture vessel at room temperature for 10 minutes. After fixation, the cells were washed three times with DPBS. Next, the cells were permeabilized by adding 0.2 % Triton X-100 solution and incubating at room temperature for 10 minutes, followed by three additional washes with DPBS. To block nonspecific binding, the cells were treated with 10 % normal goat serum at room temperature for 30 minutes. The primary antibodies (Oct4 and Nanog) were then added, and the cells were incubated overnight at 4 °C in the dark. After three washes with DPBS, secondary antibodies were added and incubated at room temperature for 1 hour. Finally, the cells were washed three more times with DPBS and observed under a fluorescence microscope.

As a result, the umbilical cord-derived adherent stem cells according to an embodiment exhibited selective positivity for CD200, CD141, CD61, CD87, and SSEA4, and selective negativity for TRA-1, CD3, CD1a, CD11c, CD16, CD86, CD8a, CD40, and MIC A/B. In addition, CD61 was selectively expressed under hypoxic conditions. Furthermore, it was confirmed that the umbilical cord-derived adherent stem cells according to the embodiment did not express the embryonic stem cell-specific markers Oct4 and Nanog.

### Experimental Example. Non-Clinical Efficacy Evaluation for Ovarian Insufficiency

### 1.Establishment of a Primary Ovarian Insufficiency (POI) Mouse Model

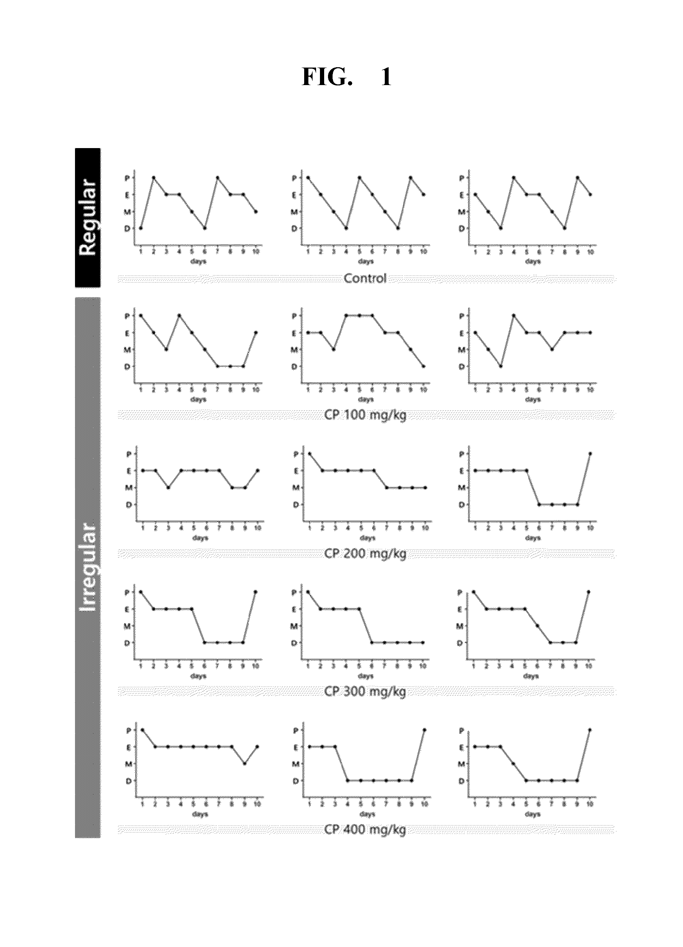

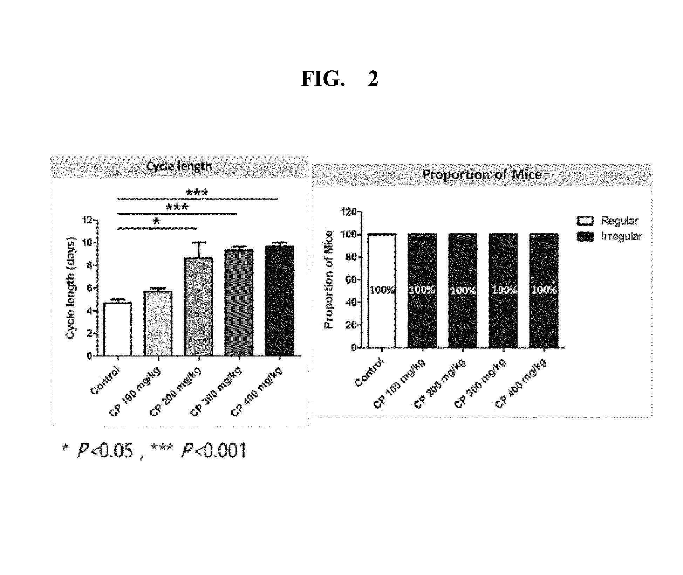

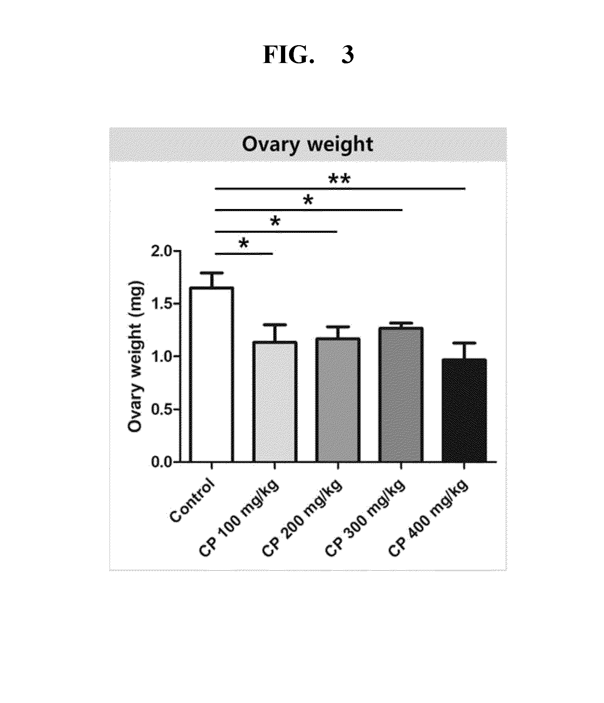

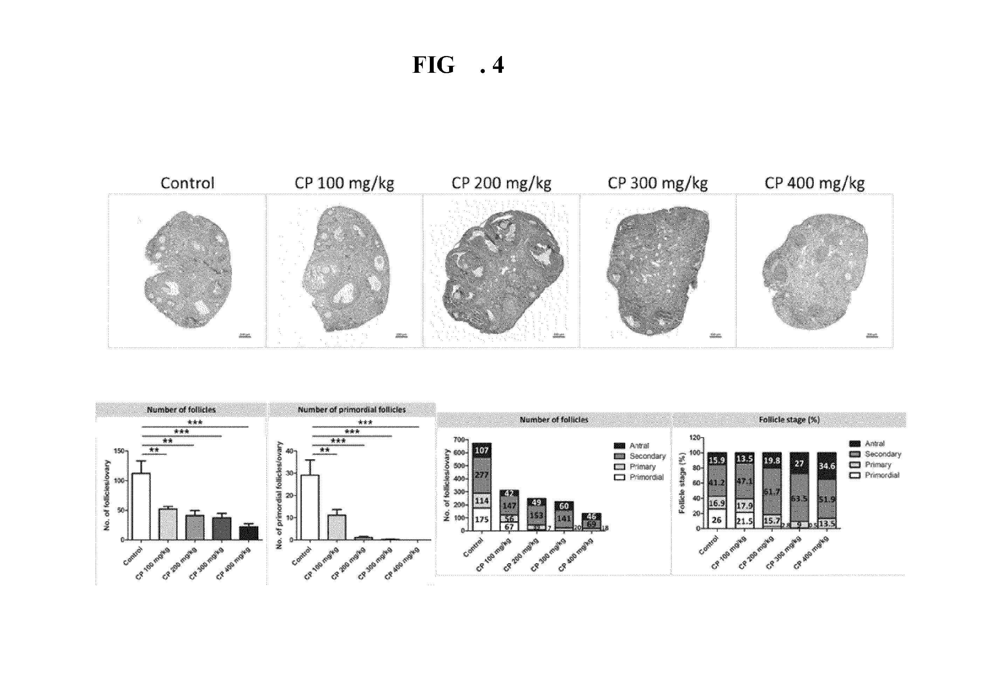

To establish a POI mouse model, 6-week-old female C57BL/6N mice were administered cyclophosphamide (CP) at doses of 100, 200, 300, or 400 mg/kg. The estrous cycle, ovarian weight, and follicle count were measured, and the results are shown in FIGS. 1 to 4.

As shown in FIGS. 1 to 4, administration of CP resulted in ovarian insufficiency. Accordingly, the 200 mg/kg CP group was selected for subsequent efficacy evaluation.

### 2. Non-Clinical Efficacy Evaluation

To evaluate the efficacy of the stem cells prepared in Example 1 for treating ovarian insufficiency, CordSTEM according to an embodiment was intravenously administered once at a dose of 100 µL/head on Day 7 after CP administration. On Day 17, the mice were euthanized, and samples were collected.

The test groups were organized as follows:
Group 1: Normal control group (N = 10)
Group 2: Sham group (CP 200 mg/kg administration, N = 10)
Group 3: Low-dose cell administration group (N = 10, CordSTEM 5.33 × 10⁴ cells/mouse)
Group 4: Medium-dose cell administration group (N = 10, CordSTEM 8 × 10⁴ cells/mouse)
Group 5: High-dose cell administration group (N = 10, CordSTEM 1.07 × 10⁵ cells/mouse)

The evaluation items are as follows.

### (1) Histological Analysis

On the scheduled necropsy day, the mice were euthanized, and both ovaries were excised. The weight of each ovary was measured, and the tissues were fixed in 4 % paraformaldehyde solution. Paraffin blocks were then prepared using the left and right ovaries. For sectioning, each ovary was serially sliced into 10 consecutive 5 µm-thick sections per part, and this was repeated until the entire ovarian tissue was sectioned. Each part was separated by a 55 µm interval. The following analyses were performed on the sections: (1-1) Hematoxylin and eosin (H&E) staining for follicle counting, (1-2) TUNEL assay for apoptosis, and (1-3) Immunohistochemistry (IHC) for Anti-Müllerian Hormone (AMH).

### (2) Superovulation Oocyte Count

On Day 7 following a single intraperitoneal administration of 200 mg/kg CP, CordSTEM was administered. On Day 21 after CordSTEM administration, 7.5 IU of pregnant mare serum gonadotropin (PMSG) was injected intraperitoneally. After 48 hours, 7.5 IU of human chorionic gonadotropin (hCG) was also administered intraperitoneally. Fifteen hours later, the oviducts of the superovulated female mice were excised. The cumulus-oocyte complexes (COCs) retrieved from the oviducts were treated with 10 µL of hyaluronidase to remove the cumulus cells, and the number of oocytes was counted.

### (3) In Vitro Fertilization: Evaluation of Fertilization Rate and Embryo Development Rate

On Day 7 following administration of the test substance, 7.5 IU of PMSG was injected intraperitoneally. After 48 hours, 7.5 IU of hCG was administered intraperitoneally. Fifteen hours later, the oviducts were excised from the superovulated female mice. Sperm was collected by squeezing the epididymis of male mice and incubated for 1 hour at 37 °C in 5 % CO₂. COCs (cumulus-oocyte complexes) were extracted from the oviducts and treated with 10 µL of hyaluronidase to remove the cumulus cells. The denuded oocytes were collected in the center of a culture dish, and 10 µL of sperm was added around the oocytes. The mixture was incubated for 4 hours at 37 °C in 5 % CO₂. Afterward, the oocytes were washed to remove all remaining sperm and cultured to observe embryo development.

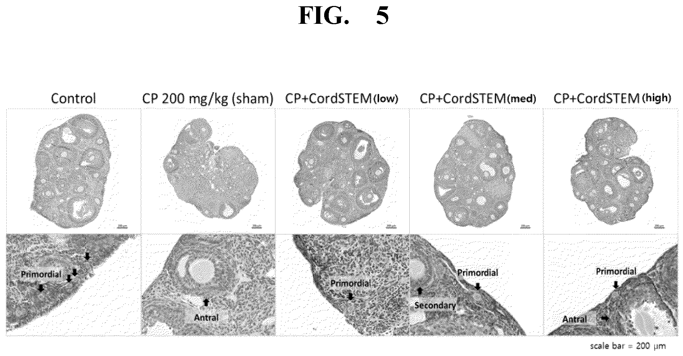

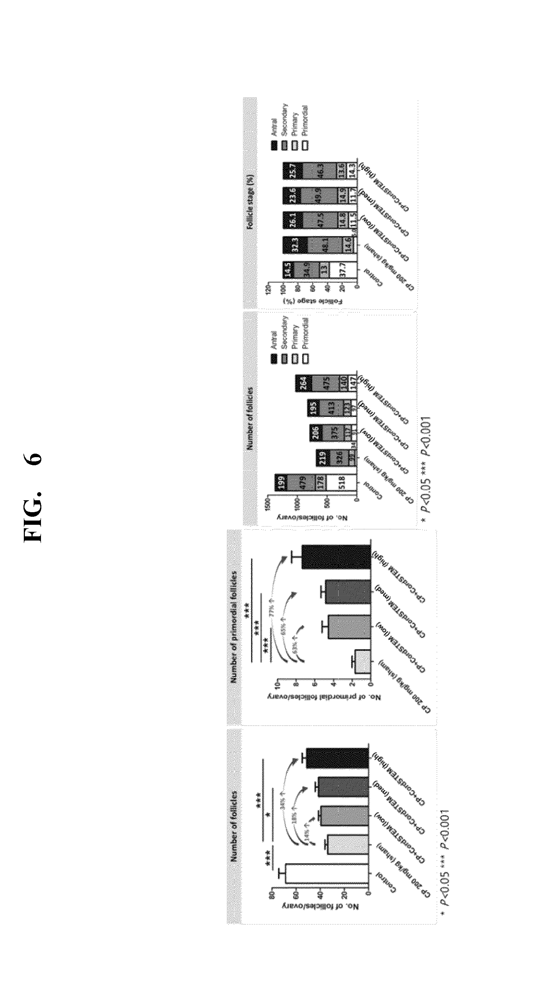

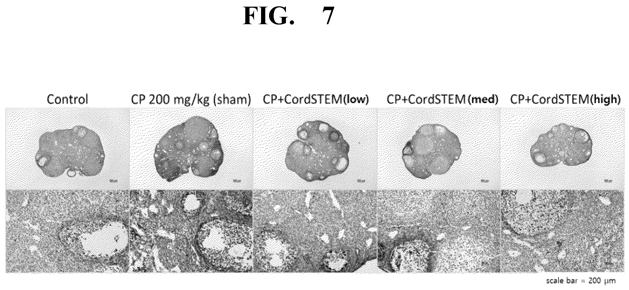

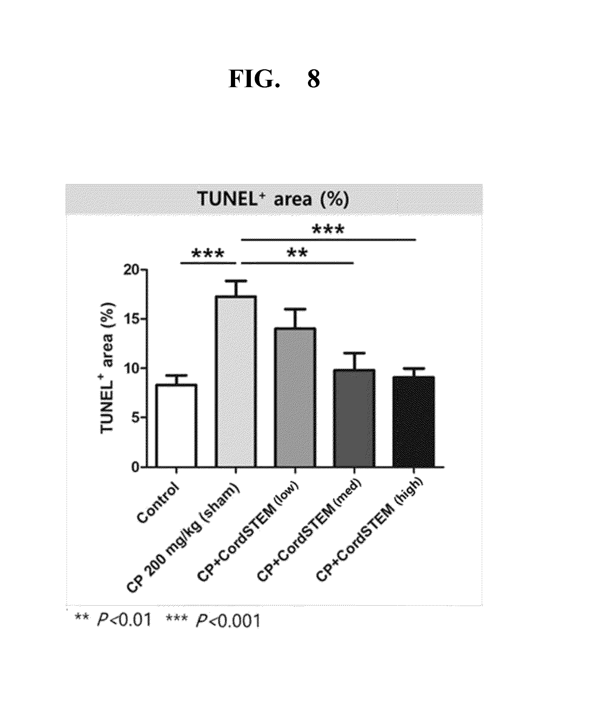

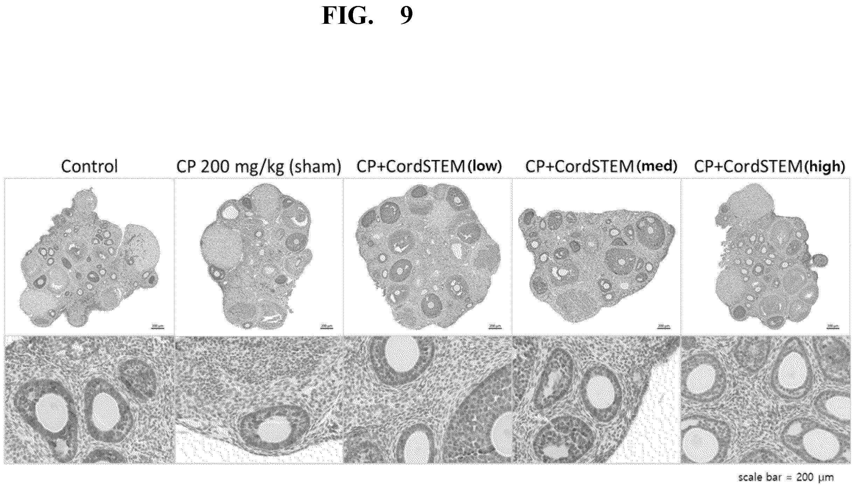

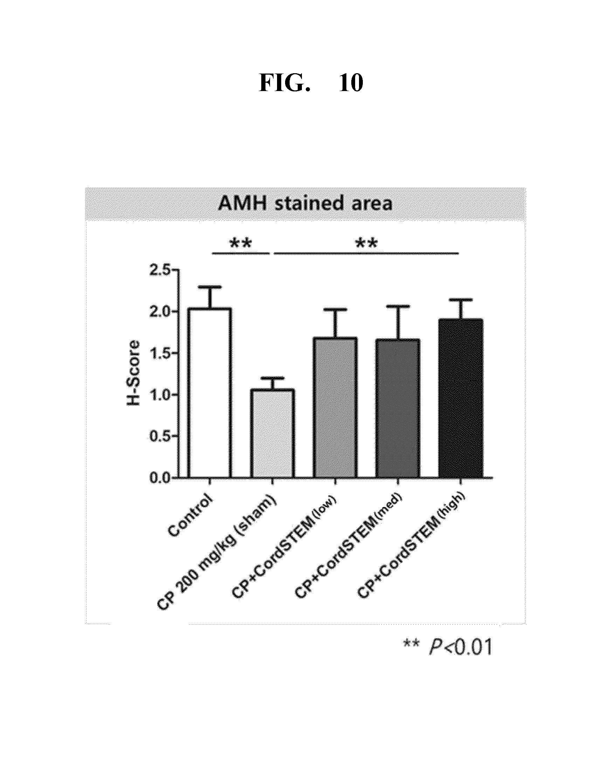

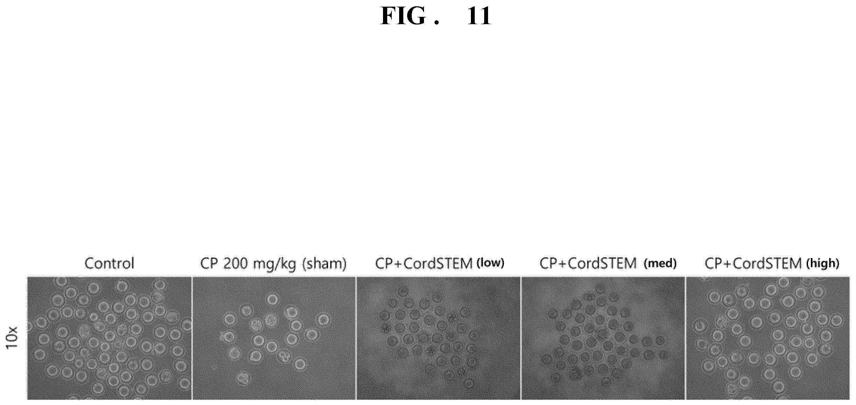

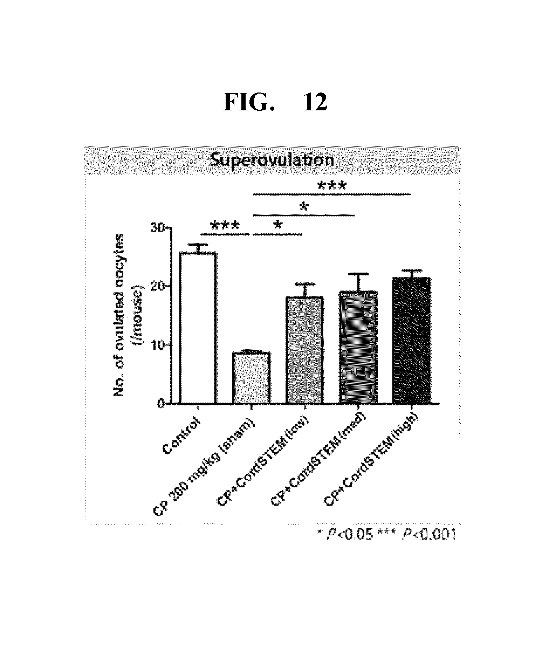

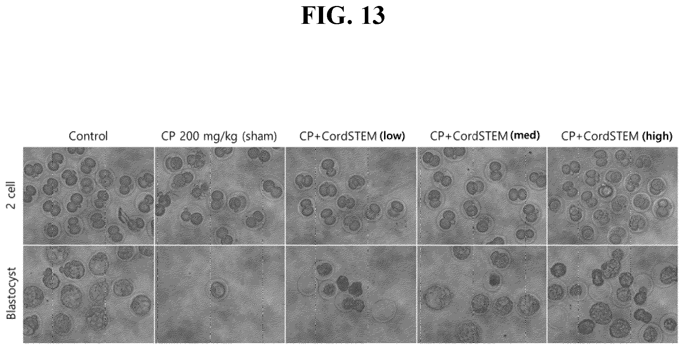

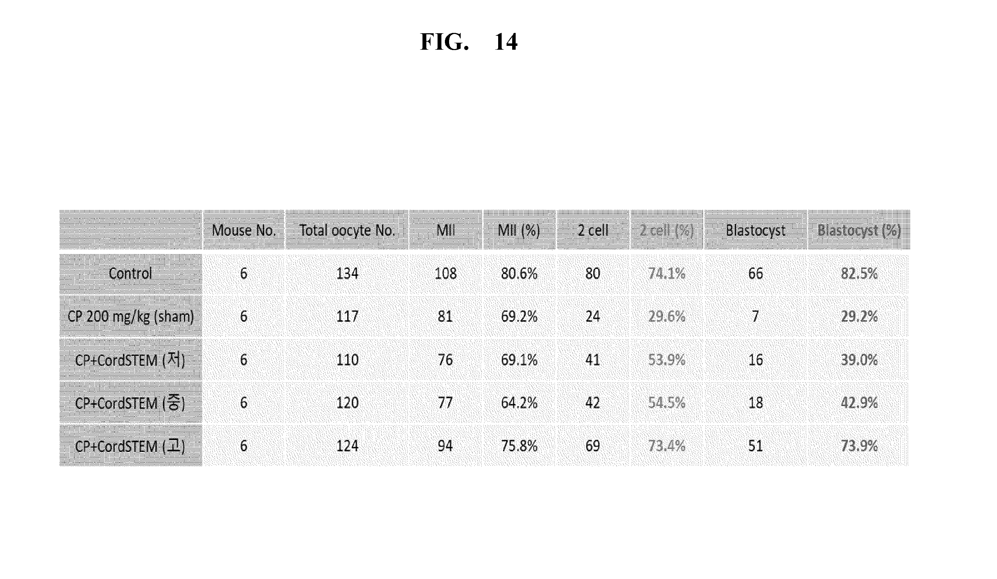

The results of (1-1) are shown in FIGS. 5 and 6; those of (1-2) are shown in FIGS. 7 and 8; those of (1-3) are shown in FIGS. 9 and 10; those of (2) are shown in FIGS. 11 and 12; and those of (3) are shown in FIGS. 13 and 14.

As shown in FIGS. 5 and 6, administration of the stem cells according to an embodiment resulted in a dose-dependent increase in the number of follicles, the proportion of follicles in the ovary, and the number of primordial follicles compared to the untreated control group.

As shown in FIGS. 7 and 8, administration of the stem cells significantly reduced ovarian apoptosis relative to the control group.

As shown in FIGS. 9 and 10, administration of the stem cells significantly increased the levels of anti-Müllerian hormone (AMH), which is associated with ovarian function, compared to the control group.

As shown in FIGS. 11 and 12, administration of the stem cells significantly increased the number of superovulated oocytes in an animal model with impaired ovarian function and reduced ovulation.

As shown in FIGS. 13 and 14, administration of the stem cells significantly enhanced the development of fertilized embryos, specifically the 2-cell and blastocyst stages, during in vitro fertilization compared to the control group.

These findings indicate that administration of the stem cells according to an embodiment may be usefully employed in the prevention or treatment of ovarian insufficiency, including premature menopause, infertility, or polycystic ovary syndrome.

### 3. Single-Cell Level Biomarker Validation

To elucidate the therapeutic mechanism of the stem cells prepared in Example 1 for treating ovarian insufficiency, biomarker analysis at the single-cell level was conducted.

Ovarian samples from the normal group, negative control group, low-dose CordSTEM administration group, and high-dose CordSTEM administration group were finely chopped using a surgical scalpel and then incubated in 0.2 % collagenase at 37 °C for 20 minutes. Collagenase activity was neutralized by adding FACS buffer (2 % FBS/PBS), followed by two rounds of centrifugation-based washing. The single cells dissociated from the ovarian tissue were then stained with FACS antibodies: PE-Cy7-conjugated anti-CD140a and Alexa488-conjugated anti-CD45. Dead cells were excluded using DAPI staining. CD140a+ cells, CD45+ cells, and remaining cells (CD140a-/CD45-) were individually sorted into wells of a 96-well plate containing lysis buffer (0.1 % Triton X-100, 1 U/µL RNase inhibitor, 0.25 µM oligo dT, and 2.5 mM dNTP). The plates were immediately snap-frozen in liquid nitrogen and stored at -80 °C.

After thawing the previously frozen 96-well plate on ice, reverse transcription was initiated by adding a reaction mixture containing 1× Maxima H- buffer, 1 M betaine, 5 µM MgCl₂, 1 µM TSO, 40 U/µL RNase inhibitor, and 200 U/µL Maxima H-RTase. A whole transcriptome amplification reaction was conducted by adding to the same 96-well plate a reagent mixture containing 1× KAPA HiFi buffer, 300 µM dNTPs, 500 µM MgCl₂, 0.167 µM and 0.1 µM ISPCR primers, and 1 U/µL KAPA HiFi DNA polymerase. The amplified cDNA was purified using Sera-Mag SpeedBeads, and quality control (QC) was performed via GAPDH qPCR. Samples that did not show a Ct value in the qPCR assay were excluded from sequencing library preparation. For library preparation, 1× homemade Tn5 buffer and Tn5 transposase were added to the single-cell cDNA libraries, followed by a 5-minute reaction at 55 °C and immediate cooling on ice. Subsequently, neutralize tagment buffer was added and the mixture was left at room temperature for 5 minutes. NEXTERA indexes and a sequencing library amplification reagent mixture (1× KAPA buffer, 300 µM dNTPs, 500 µM MgCl₂, 1 U/µL KAPA enzyme) were then added to carry out the amplification. After completing the reaction, all samples were pooled, and 5× the volume intended for sequencing was added as PB (binding buffer). The PCR products were then purified using a standard purification kit. The purified DNA was quantified and analyzed using a TapeStation system. To prepare the sequencing library, 0.3× volume of magnetic beads was added to the sample, followed by magnetic separation. The supernatant was collected, and 0.6× volume of magnetic beads was added for a second magnetic separation. After removing the supernatant, the pellet was washed with 80 % ethanol, and residual ethanol was completely removed. EB buffer was then added, and magnetic separation was performed again to elute the library. The sequencing library was stored at 4 °C. The sequencing library was subjected to paired-end sequencing using a NextSeq 550 platform.

Next, post-sequencing reads were aligned to the mm10 mouse transcriptome using the STAR aligner, and sample-specific TPM (Transcripts Per Million) and raw counts were calculated using RSEM. TPM values were transformed using log₂(TPM+1), and the analysis was limited to cells expressing more than 500 genes and genes expressed in at least five different cells. The analysis was conducted in a Python environment using the Numpy, SciPy, Matplotlib, scikit-learn, and pandas packages. Variable genes were selected, followed by principal component analysis (PCA) and t-distributed stochastic neighbor embedding (t-SNE) visualization based on the selected genes. For cell population analysis, unsupervised hierarchical clustering was performed. Differentially expressed genes and marker genes were identified using the DESeq2 package in an R software environment.

Quantitative results were statistically analyzed using Prism Version 5.03. Student's t-test was performed to compare the normal group and the negative control group, and one-way ANOVA was used to compare the negative control group with the test substance-treated groups. Statistical significance was determined at p < 0.05.

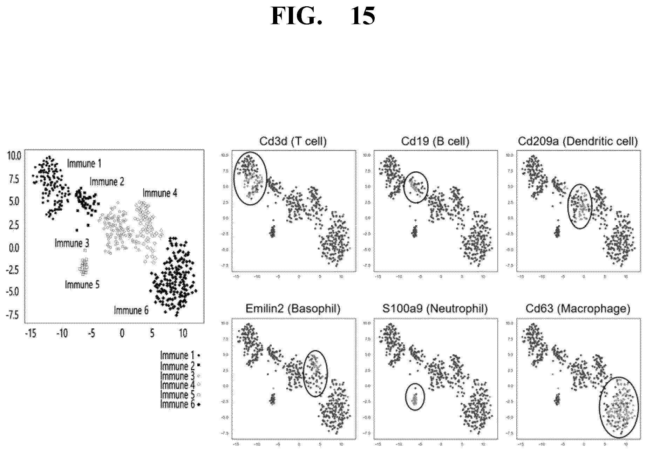

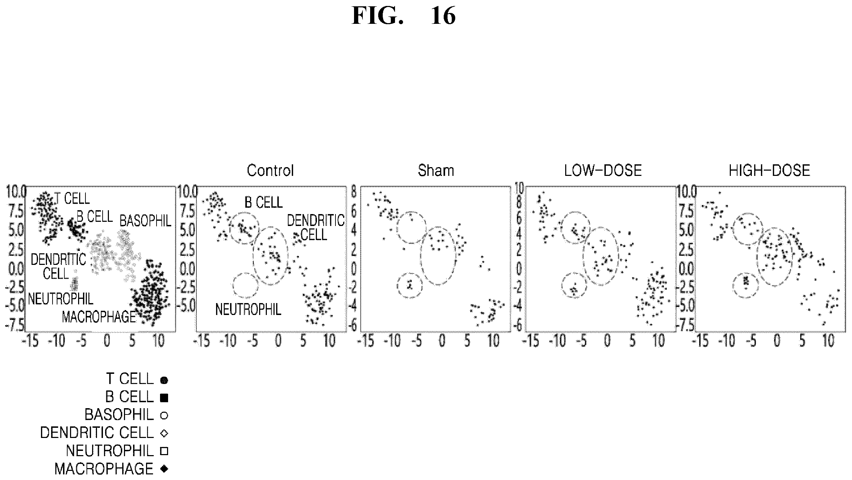

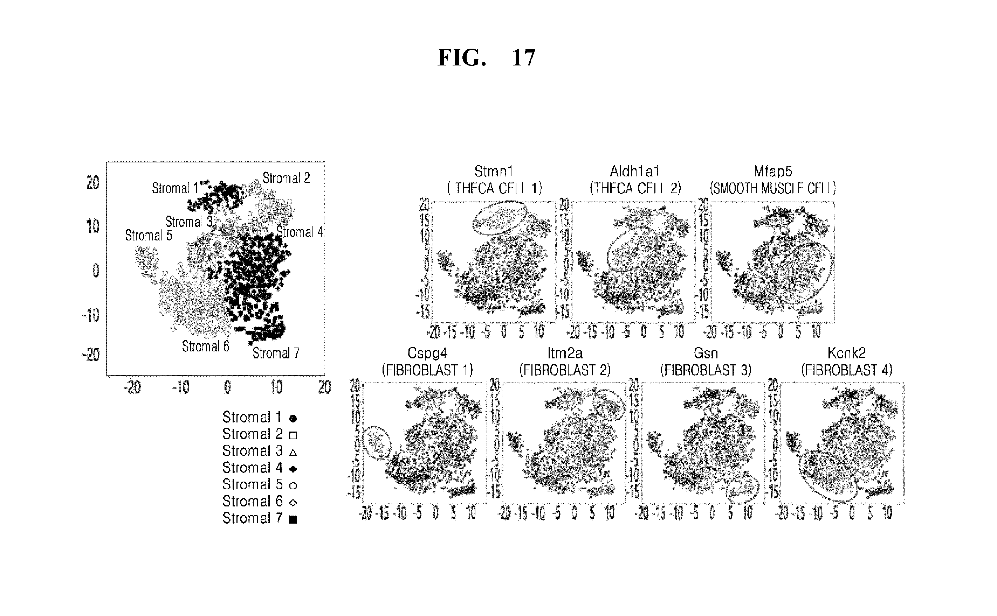

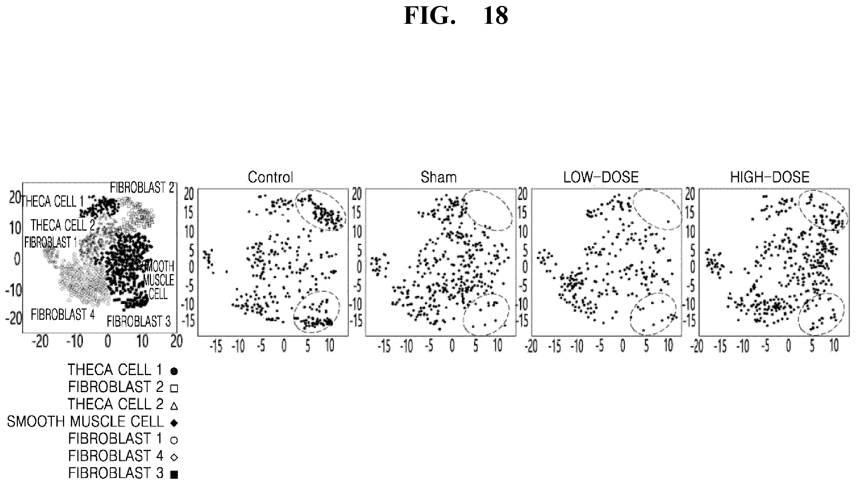

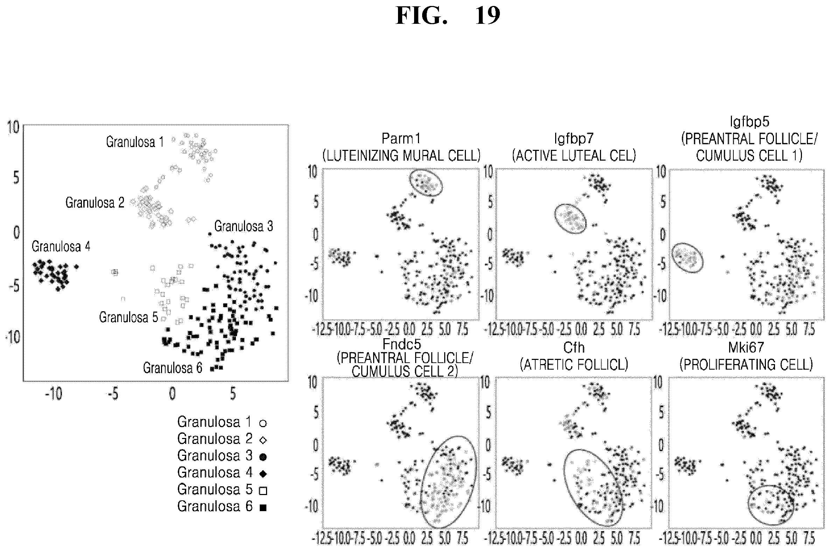

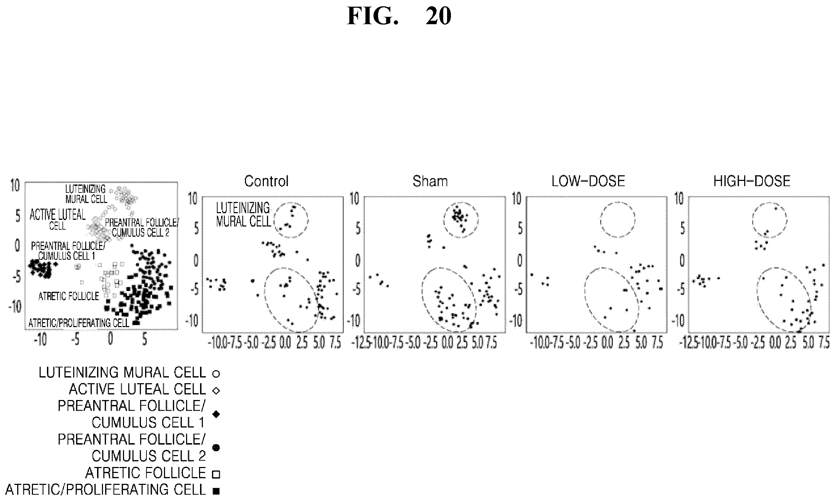

The results of immune cell population analysis are shown in FIGS. 15 and 16, those of stromal cell populations in FIGS. 17 and 18, and those of granulosa (GC) cell populations in FIGS. 19 and 20.

As shown in FIGS. 15 and 16, in POI-induced mice treated with CP, the reduced B cell and dendritic cell populations were restored following administration of the stem cells according to an embodiment. In particular, in the high-dose cell administration group, the dendritic cell population recovered to levels comparable to the normal group. These findings suggest that recovery of ovarian function by the stem cells according to an embodiment may occur via restoration of dendritic cells residing in the ovary.

As shown in FIGS. 17 and 18, in POI-induced mice treated with CP, the reduced Fibroblast 3 population (Gsn) was not restored even after administration of the stem cells according to an embodiment. However, the Fibroblast 2 population (ltm2a) was restored following administration of the stem cells.

As shown in FIGS. 19 and 20, the increased luteinizing mural cell population (Parm1) and atretic cell population (Cfh) in CP-treated POI-induced mice were decreased following administration of the stem cells according to an embodiment. Notably, the luteinizing mural and atretic cell populations recovered to levels comparable to those of the normal control group. Considering that atretic cells represent degenerating follicles that fail to develop properly, the reduction in atretic cells by the administered stem cells demonstrates a significant therapeutic effect on ovarian insufficiency.

Based on the above results, it was confirmed that the stem cells according to an embodiment restored the function of B cells, dendritic cells, fibroblasts, luteinizing mural cells, and atretic cells in the POI mouse model. These findings suggest that the stem cells according to an embodiment may promote recovery of the supporting somatic cell populations essential for oocyte development and maturation, thereby contributing to the restoration of ovarian function.

## Claims

1. A pharmaceutical composition for preventing or treating ovarian insufficiency, comprising umbilical cord-derived adherent stem cells as an active ingredient.

2. The pharmaceutical composition of claim 1, wherein the umbilical cord-derived adherent stem cell has one or more surface antigen characteristics selected from the group consisting of D200+, Tra-1-60-, CD3-, CD1a-, CD11c-, CD16-, CD86-, CD8a-, CD40-, CD141+, CD61+, CD87+, MIC A/B-, and SSEA4+.

3. The pharmaceutical composition of claim 1, wherein the ovarian insufficiency is any one selected from primary ovarian insufficiency, secondary ovarian insufficiency, hyperandrogenemia, polycystic ovary syndrome, amenorrhea, oligomenorrhea, premature menopause, and infertility.

4. The pharmaceutical composition of claim 1, wherein the stem cells increase the number of follicles or primordial follicles.

5. The pharmaceutical composition of claim 1, wherein the stem cells promote ovulation of oocytes.

6. The pharmaceutical composition of claim 1, wherein the stem cells promote development of fertilized embryos.

7. The pharmaceutical composition of claim 1, wherein the stem cells restore the function or activity of any one cell selected from the group consisting of B cells, dendritic cells, and fibroblasts, which are decreased in ovarian insufficiency.

8. The pharmaceutical composition of claim 1, wherein the stem cells decrease the number or activity of luteinizing mural cells or atretic cells, which are increased in ovarian insufficiency.

9. The pharmaceutical composition of claim 1, wherein the stem cells increase the expression or activity of CD19 or CD209a.

10. The pharmaceutical composition of claim 1, wherein the stem cells increase the expression or activity of Itm2a, or decrease the expression or activity of Parm1 or Chf.

11. The pharmaceutical composition of claim 1, wherein the stem cells are administered in an amount of 1.0 × 10³ to 1.0 × 10¹⁰ cells per subject.

12. A method of preventing or treating ovarian insufficiency, comprising administering an effective amount of umbilical cord-derived adherent stem cells to a subject in need thereof.

13. A method of increasing the number of follicles or primordial follicles in a subject, comprising administering an effective amount of umbilical cord-derived adherent stem cells to the subject in need thereof.

14. A method of promoting ovulation of oocytes in a subject, comprising administering an effective amount of umbilical cord-derived adherent stem cells to the subject in need thereof.

15. A method of promoting the development of fertilized embryos in a subject, comprising administering an effective amount of umbilical cord-derived adherent stem cells to the subject in need thereof.

16. A method of increasing or decreasing, respectively, the function or activity of cells that are decreased or increased in ovarian insufficiency, the method comprising administering an effective amount of umbilical cord-derived adherent stem cells to a subject in need thereof.

17. The method of claim 16, wherein the decreased cells in ovarian insufficiency are any one cell selected from the group consisting of B cells, dendritic cells, and fibroblasts.

18. The method of claim 16, wherein the increased cells in ovarian insufficiency are luteinizing mural cells or atretic cells.

19. Use of umbilical cord-derived adherent stem cells for the manufacture of a pharmaceutical preparation for preventing or treating ovarian insufficiency.
